# EUROPÄISCHE PATENTSCHRIFT

(11) **EP 0 443 599 B1**
(45) Veröffentlichungstag und Bekanntmachung des Hinweises auf die Patenterteilung: **29.09.1999**
(21) Anmeldenummer: 91102608.6
(22) Anmeldetag: 22.02.1991
(51) Int. Cl.: G01N 33/574, G01N 33/577

(54) **Nachweisverfahren von tumorassoziierten Antigenen**
method for the detection of tumour associated antigens
Méthode pour la détection des des antigènes associés aux tumeurs

(30) Priorität: 22.02.1990 DE 4005630
(43) Veröffentlichungstag der Anmeldung: 28.08.1991
(60) Teilanmeldung: 99103484.4
(73) Patentinhaber: Dade Behring Marburg GmbH, 35001 Marburg (DE)
(72) Erfinder: Bosslet, Klaus, Dr., W-3550 Marburg (DE); Auerbach, Bernhard, Dr., W-3550 Marburg (DE); Peters, Helmut, Dr., W-3550 Marburg (DE)

(56) Entgegenhaltungen:
- JOURNAL OF PATHOLOGY, Band 159, Nr. 1, September 1989, Seiten 23-28, By John Wiley & Sons., Ltd, Edinburgh, GB; R.E. KIBBELAAR et al.: "Expression of the embryonal neural cell adhesion molecule N-cam in lung carcinoma. Diagnostic usefulness of monoclonal antibody 735 for the distinction between small cell lung cancer and non-small cell lung cancer"
- CANCER RESEARCH, Band 47, Nr. 3, 1 Februar 1987, Seiten 826-829, Philadelphia, US; J-I. WATANABE et al.: "Monoclonal antibody that distinguishes small-cell lung cancer from non-small-cell cancer"
- CANCER RESEARCH, Band 50, Nr. 4, 15. Februar 1990, Seiten 1102-1106, Philadelphia, US; C.E.C.K. MOOLENAAR et al.: "Expression of neural cell adhesion molecule-related sialoglycoprotein in small cell lung cancer and neuroblastoma cell lines H69 and CHP-212"
- BIOTECHNOLOGY AND APPLIED BIOCHEMISTRY, Band 10, Nr. 3, Juni 1988, Seiten 273-286, Academic Press., Inc., Orlando, Fla., US; B.B. FUCHS et al.: "Biochemical and immunochemical analysis of gangliosides of human small cell lung carcinoma: Production of monoclonal antibodies against a unique marker of small cell lung carcinoma, ganglioside Fuc GM"

## Beschreibung

Die Erfindung betrifft Verfahren zum qualitativen Nachweis und zur quantitativen Bestimmung eines tumorassoziierten Antigens aus der Klasse der als N-CAM bezeichneten Glykoproteine in menschlichen Körperflüssigkeiten, dadurch gekennzeichnet, daß ein spezifischer Bindungspartner, der gegen Sialinsäure, Polysialinsäure oder α-(2,8)-verknüpfte N-Acetyl-Neuraminsäure gerichtet ist, in Kombination mit einem zweiten spezifischen Bindungspartner eingesetzt wird, der nicht gegen Sialinsäure, Polysialinsäure oder α-(2,8)-verknüpfte N-Acetyl-Neuraminsäure gerichtet ist, sondern ein anderes Epitop auf dem tumorassoziierten Antigen erkennt.

Die Identifizierung, Charakterisierung und Therapie von Tumoren gehört zu den wichtigsten Gebieten der Diagnostik und Therapie. Durch die Möglichkeit, monoklonale Antikörper hoher Spezifität zu produzieren, hat die Entwicklung auf diesem Gebiet große Fortschritte gemacht. Als besonders wichtig hat sich dabei die Identifizierung von sogenannten Tumormarkern erwiesen. Unter Tumormarker versteht man Produkte der Tumorzelle, z. B. tumorassoziierte Antigene, aber auch Substanzen, die vom gesunden Gewebe als Reaktion des Organismus auf das maligne Wachstum gebildet werden. Bekannte Tumormarker sind z. B. das CEA, das AFP, aber auch durch monoklonale Antikörper definierte Tumorantigene, wie z. B. das CA 19-9 oder das CA 125.

Das Haupteinsatzgebiet der Tumormarker in der In-vitro-Diagnostik besteht in der Therapie sowie der Verlaufskontrolle bei Tumorpatienten. Bestimmte Tumormarker können auch für die Differentialdiagnose oder für das Screening von Risikogruppen eingesetzt werden.

Zur Identifizierung von kleinzelligen Bronchialkarzinom (SCLC) sind schon eine Reihe von Versuchen unternommen worden. So ist zum Beispiel bekannt, daß die Neuron-spezifische Enolase, ein Isoenzym der Enolase (EC 4.2.1.11), von malignen Tumoren neuroektodermalen Ursprungs, wie z. B. dem kleinzelligen Bronchialkarzinom oder dem Neuroblastom vermehrt gebildet wird und bei Tumorpatienten in erhöhten Konzentrationen im Serum auftritt.

Es hat sich aber gezeigt, daß ein Teil der an den o.g. Tumoren erkrankten Patienten falsch-negativ erfaßt wird. Da rote Blutkörperchen, aber auch Thrombozyten, größere Mengen an NSE enthalten, ist es ferner der Fall, daß durch deren Lyse falsch erhöhte NSE-Serum- oder Plasmaspiegel und somit falsch-positive Werte gemessen werden (Pahlman et al., Tumour Biology 5: 127 - 139, 1984).

Aus der Patentanmeldung EP 0 323 802 ist ein monoklonaler Antikörper gegen ein Zelloberflächenantigen von Lungenkarzinomen bekannt. Aus MAIER et al. (Br.J. Cancer, 1989, 59, 692 - 695) ist aber bekannt, daß der in der EP 0 323 802 verwendete Antikörper SWA 20 ein Epitop (Cluster 5) erkennt, das nur bei 45 % der untersuchten SCLC-Proben eine mittel bis stark positive Reaktion zeigte.

Es ist daher wünschenswert, einen anderen von der NSE unabhängigen, spezifischen Tumormarker für das Neuroblastom bzw. das kleinzellige Lungenkarzinom zu produzieren.

Die Erfindung betrifft Nachweisverfahren zur Bestimmung eines tumorassoziierten Antigens. Hierzu werden monoklonale Antikörper gegen ein tumorassoziiertes Antigen vorgeschlagen, wobei das Antigen von Tumoren vornehmlich aus der Gruppe der neuroektodermalen Tumoren, wie z. B. dem kleinzelligen Lungenkarzinom (SCLC), dem Melanom, dem Neuroblastom sowie aus dem Kulturüberstand von Zellinien aus diesen Tumoren stammt, insbesondere Antigene aus SCLC-Zellinien, die in der nichtreduzierenden SDS-PAGE ein Molekulargewicht von 170 ± 10 kDa, 140 ± 10 kDa, 105 ± 10 kDA, 67 ± 10 kDa und 50 ± 10 kDA aufweisen oder daß das Antigen aus Körperflüssigkeiten von Tumorpatienten stammt, insbesondere Antigene aus dem Serum von SCLC-Patienten, die in der nichtreduzierenden SDS-PAGE Molekulargewichte von 220-260 kDa und 160-200 kDa aufweisen. Diese Banden werden mit dem MAK BW SCLC-1 im Western blot nachgewiesen und bei 5 von 5 SCLC-Tumorpatienten gefunden. Bei 2 von 4 Normalseren wurde keine Bande, bei 2 ganz schwache Banden in der gleichen Position wie in den Tumorseren gefunden.

Bevorzugt sind dabei solche monoklonalen Antikörper, die an ein Antigen binden, das auch durch die Referenzantikörper MAK BW SCLC-1 und/oder MAK BW SCLC-2 gebunden wird.

Besonders bevorzugt sind dabei solche monoklonalen Anti-Körper, die von mindestens einer der Hybridomazellinien BW SCLC-1 und SCLC-2 produziert werden.

Unter monoklonalen Antikörpern werden im Sinne dieser Erfindung auch Antikörperfragmente, wie z. B. Fab und F(ab)₂ und Derivate verstanden.
Die Hybridomazellinien BW SCLC-1 und 2, die die monoklonalen Antikörper MAK BW SCLC-1 und MAK BW SCLC-2 produzieren, sind unter der Nummer 90 022 110 bzw. 90 022 109 am 21. Februar 1990 bei der European Collection of Animal Cell Cultures (ECACC) hinterlegt worden.

Die Erfindung betrifft ferner die Verwendung spezifische Bindungspartner, wie z. B. mono- oder polyklonale Antikörper, Lectine und ähnliche Substanzen, die sich dadurch auszeichnen, daß sie an dieselben Antigene binden können, wie die Referenzantikörper.
Referenzantikörper im Sinne der Erfindung sind die MAK BW SCLC-1 und SCLC-2.

Monoklonale Antikörper können nach dem Fachmann an sich bekannten Verfahren hergestellt werden, wobei bevorzugterweise Antigene aus dem Überstand von SCLC-Zellinien, die in der nichtreduzierenden SDS-PAGE ein Molekulargewicht von 170 ± 10 kDa, 140 ± 10 kDa, 105 ± 10, 67 ± 10 kDa oder 50 ± 10 kDa aufweisen, zum Immunisieren verwendet werden. Antigene können durch Immunadsorption an einen der vorgeschlagenen Antikörper gebunden werden.

Unter Immunadsorption im Sinne dieser Beschreibung verstehen wir dem Fachmann an sich bekannte Isolierungsverfahren, bei denen mindestens ein Reinigungsschritt auf einer immunchemischen Reaktion mit einem der vorgeschlagenen Antikörper beruht. Die Abtrennung des Ak-Ag Komplexes kann dabei auf dem Fachmann an sich bekannte Weise, z. B. durch Bindung des Antikörpers an eine Festphase erfolgen.

Die Erfindung betrifft die Verwendung der vorgeschlagenen Antikörper und/oder Antigene in der Diagnostik.

In der Diagnostik werden bevorzugt Antikörper in dem Fachmann an sich bekannten immunchemischen heterogenen oder homogenen Bestimmungsmethoden eingesetzt, wobei bei den homogenen Verfahren die Partikel-verstärkte Nephelometrie oder Turbidimetrie bevorzugt ist. Bei den heterogenen Immunoassays wird der festphasengebundene Sandwichassay bevorzugt, wobei die Festphase bevorzugterweise ein Polystyrolröhrchen, ein Latexpartikel, ein magnetisierbarer Partikel oder eine flächenförmige Festphase ist.
Bevorzugt ist ein diagnostisches Verfahren zum Nachweis eines tumorassoziierten Antigens, wobei ein vorgeschlagenen Antikörper als spezifischer Bindungspartner eingesetzt wird.

Die vorgeschlagenen Antikörper und Antigene können auch in Biosensoren eingesetzt werden. Biosensoren sind dem Fachmann an sich bekannt. Besonders bevorzugt ist ein Verfahren, wobei ein zweiter spezifischer Bindungspartner, wie z. B. ein Antikörper, ein Lectin oder ein Rezeptor eingesetzt wird.

Ganz bevorzugt ist dabei ein Verfahren, wobei der zweite spezifische Bindungspartner Sialinsäure, Polysialinsäure oder α(2-8)-verknüpfte N-Acetyl-Neuraminsäure spezifisch erkennt.

Zum Nachweis und zur Quantifizierung kann dabei einer der spezifischen Bindungspartner ein nachweisbares Label tragen. Diese Label sind dem Fachmann an sich bekannt und können z. B. ein Chromophor, ein Luminophor, ein Fluorophor, ein Enzym, ein radioaktives Isotop oder ein gefärbter oder auch ungefärbter Partikel sein.
Bevorzugt ist ein Verfahren, wobei der nicht-markierte spezifische Bindungspartner, nach dem Fachmann an sich bekannten Verfahren, direkt oder indirekt, z. B. über einen weiteren Antikörper oder eine Biotin-Avidin Brücke an eine Festphase gekoppelt ist.

Besonders bevorzugt sind ferner die in den Beispielen beschriebenen Ausführungsformen.

Die MAK BW SCLC-1 und -2 können aufgrund ihrer immunhistochemischen Bindung an menschliche Normalgewebe und Tumore als SCLC cluster 1 MAK bezeichnet werden (Souhami et al., LANCET 8. August 1987, S. 325 - 326). Dieses Cluster enthält MAK, die optimal an kleinzellige Lungenkarzinome binden. Desweiteren binden diese MAK an neurales Gewebe, Neuroblastome und einige Melanome. In Moolenaar et al. (Cancer Res., 1990, 50, 1102-1106) wurde die Bindung verschiedenen SCLC cluster 1 MAK an deren Epitope untersucht.

Patel et al. (Int. J. Cancer 44: 573 - 578, 1989) haben gezeigt, daß diese Cluster 1 MAK N-CAM erkennen und zwar hauptsächlich die 140 und 180 kDa Isoformen (Patel et al., Int. J. Cancer 44: 1062 - 1068, 1989). Es ist bisher noch nicht beschrieben worden, daß das N-CAM und insbesondere die 160 - 180, 130 - 150, 95 - 115 , 57 - 77 bzw. 40 - 60 kDa (Kulturüberstand) sowie die 220 - 260 kDa und 160 bis 200 kDa (Serum) iso-Form von Tumorzellen aktiv ausgeschieden werden und somit als Tumormarker genutzt werden können. Da das N-CAM u. a. auch im Nerven-, Muskel- und Nierengewebe nachweisbar ist (Roth et al., Proc.Natl.Acad.Sci, USA 85, 2999 - 3003, 1988; Roth et al., Virchows Archiv B 56 , 95 - 102, 1988), kann erwartet werden, daß es auch bei anderen krankhaften Prozessen, insbesondere diese Gewebe betreffend, zu Veränderungen der N-CAM-Konzentration in den Körperflüssigkeiten der Patienten kommen kann, so daß N-CAM auch für diese Krankheiten als diagnostischer Marker dienen kann.

Die Spezifität der MAK BW SCLC-1 und -2 läßt sich nicht nur für eine immunhistochemische Differenzierung von verschiedenen Tumorgeweben und Normalgeweben nutzen, sondern überraschenderweise erwies sich eine Kombination eines Anti-N-CAM-MAKs als Festphasenantikörper der α-(2-8)-verknüpfte N-Acetyl-Neuraminsäure (Finne et al., J. Immunol. 138: 4402 - 4407, 1987; Häyrinen et al., Molecular Immunology 26: 523 - 529, 1989; Kibbelaar et al., J. Path 159: 23-28, 1989) erkennt, mit den MAK BW SCLC-1 und -2 als Konjugatantikörpern als besonders gut geeignet, um einen Tumormarker-Immunoassay zu entwickeln. Mit diesem Assay wurde nachgewiesen, daß die erkannten Antigene im Serum bzw. Plasma von Patienten mit SCLC und Neuroblastom häufig in einer deutlich gegenüber Serum oder Plasma gesunder Kontrollpersonen erhöhten Konzentration vorliegen. Hieraus läßt sich eine gute Sensitivität des Testes für die erwähnten Tumore ableiten.

Die Antikörper BW SCLC-1 und -2 oder deren Fragmente können auch nach dem Fachmann bekannten Verfahren radioaktiv markiert werden, um sie für die Immunszintigraphie bzw. auch für die Immuntherapie einzusetzen. Zusätzlich könnten diese monoklonalen Antikörper als Wirkstoffträger, z. B. für Zelltoxine, eingesetzt und zur Therapie maligner Erkrankung genutzt werden. Auch die Erzeugung von Antikörperkonstrukten, z. B. durch Einsetzen der hypervariablen Regionen in ein humanes MAK-Gerüst, ist nach der Analyse der vollständigen Nukleotidsequenz der V-Gene der MAK BW SCLC-1 und -2 technisch möglich (Jones et al., Nature 321: 522 - 525, 1986; Verhoeyen et al., Science 239: 1534 - 1536, 1988).

Die folgenden Beispiele dienen der Erläuterung der Erfindung und schränken sie in keiner Weise ein.

### Beispiel 1

### Erzeugung der monoklonalen Antikörper BW SCLC-1 und -2

Als Immunogen wurden die humanen kleinzelligen Lungenkarzinom-zell-Linien GOT und MR 22 benutzt. Ihre Kultivierung in vitro erfolgte als Suspensionskultur in Basalmedium (DMEM), welches mit 10 % Rinderserum versetzt ist. Balb/c-Mäuse wurden mit 3 x in Salzlösung (PBS) gewaschenen Zellen entsprechend dem nachfolgenden Schema immunisiert:

| Injektionstag | Zellzahl/Maus | Route | Adjuvans | Zelltyp |
|---|---|---|---|---|
| 0 | 1,5 x 10⁷ | S.C. | CFA | MR 22 |
| 7 | 1 x 10⁷ | S.C. | CFA | GOT |
| 14 | 1 x 10⁷ | S.C. | IFA | MR 22 |
| 21 | 1 x 10⁷ | S.C. | IFA | GOT |
| 28 | 1 x 10⁷ | S.C. | IFA | MR 22 |
| 32 | 2 x 10⁶ | i.v. | PBS | GOT |
| 33 | 2 x 10⁶ | i.v. | PBS | MR 22 |
| (Abkürzungen: CFA = komplettes Freund's Adjuvans, IFA = inkomplettes Freund's Adjuvans S.C. = subkutan i.v. = intravenös) | | | | |

Die Milzen der so immunisierten Mäuse wurden am Tag 35 entnommen und in einem Verhältnis von 6 : 1 (Milzzellen zu Myelomzellen) mit der SP-2 Myelomzell-Linie (Shulman et al., Nature 276: 269, 1978) nach der von Köhler und Milstein beschriebenen Technik fusioniert (Köhler u. Milstein, Nature 256: 495, 1975).

Die in dem Zeitraum von 8 - 28 Tagen ausgewachsenen Hybride wurden mittels zytofluorometrischer Analyse getestet, ob sie MAK ausscheiden, die an die GOT und die MR 22 SCLC-Zelllinien binden. Positive Hybride wurden mittels der limited dilution Technik 3 x kloniert und die von diesen Subklonen produzierten MAK verschie-denen immunologischen Testverfahren unterworfen. Hybride, die aufgrund dieser immunologischen Tests besonders interessante MAK ausschieden, wurden in flüssigem Stickstoff eingefroren und unter der Bezeichnung BW SCLC-1 bzw. BW SCLC-2 bei der ECACC unter der Deposit-No. 90 022110 bzw. 90 022 109 hinterlegt. Die von diesen Hybriden ausgeschiedenen MAK werden als MAK BW SCLC-1 oder MAK BW SCLC-2 bezeichnet.

### Beispiel 2

### Immunhistochemische Charakterisierung der Spezifität der MAK BW SCLC-1 und MAK BW SCLC-2

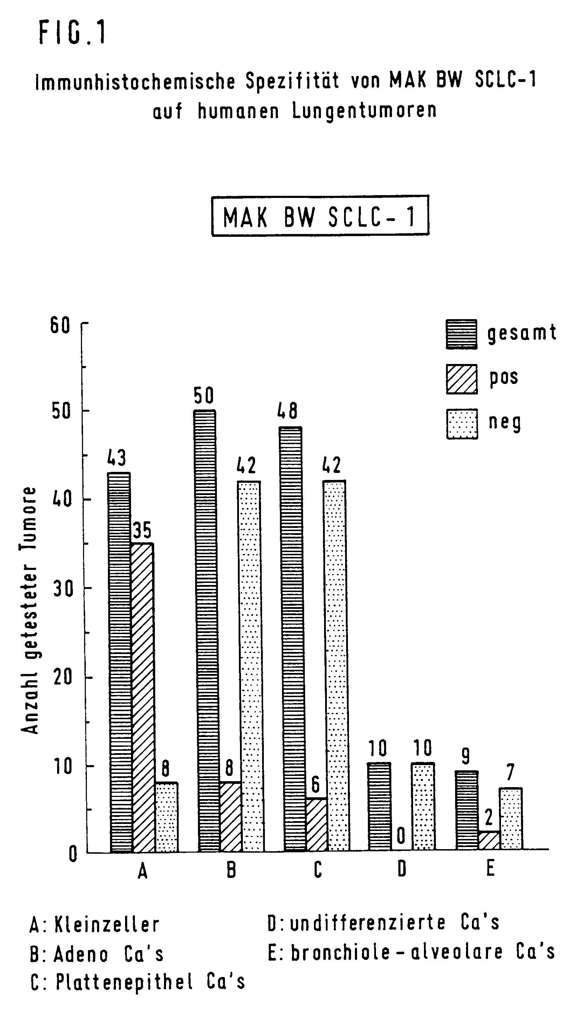

Mittels der APAAP-Technik (Cordell et al., J. Histochem. Cytochem. 32: 219, 1984) wurde die Expression der Epitope, die von beiden MAK erkannt wurden, auf kryopräservierten menschlichen Normalgeweben und Tumoren bestimmt. Hierbei stellte sich heraus, daß die Expression auf Tumore neuroektodermalen Ursprungs beschränkt ist, d. h. mehr als 80 % der kleinzelligen Lungentumore (Fig. 1), Neuroblastome und Hirntumore waren deutlich positiv (Tab. 1), zusätzlich ein Großteil der Melanome. Die meisten anderen nicht vom Neuroektoderm abgeleiteten Tumore waren negativ (s. Tab. 1). Die Reaktionen des MAK BW SCLC-1 mit menschlichen kryopräservierten Normalgeweben sind in Tab. 2 zusammengefaßt. Der MAK BW SCLC-2 zeigt ein vergleichbares Reaktionsmuster. Lediglich seine Bindung an Knochenmark ist stärker ausgeprägt.

### Beispiel 3

### Charakterisierung der von den MAK BW SCLC-1 und MAK BW SCLC-2 erkannten Antigene und Epitope

Der MAK BW SCLC-1 wurde über Protein-A affinitätschromatographisch gereinigt und ebenso wie der gegen α-(2-8)-verknüpfte N-Acetyl-Neuraminsäure gerichtete MAK 735 an CN-Br aktivierte Sepharose gekoppelt (Ey et al., Immunochemistry 15: 429, 1978; Pharmacia Fine Chemicals, Affinity Chromatography, Principles and Methods, Seite 15 - 18, 1979).
Zellkulturmedien, in denen die GOT Zell-Linie kultiviert wurde, wurden über die mit MAK BW-SCLC-1 beladene CN-Br aktivierte Sepharosesäule gepumpt und das spezifisch bei pH 7 gebundene Antigenmaterial bei pH 2,5 eluiert. Das so erhaltene Eluat wurde mittels SDS-Polyacrylamidgelelektrophorese (SDS-PAGE) sowohl unter reduzierenden als auch nicht reduzierenden Bedingungen aufgetrennt, anschließend nach dem Fachmann bekannten Verfahren einer Silberfärbung unterzogen bzw. auf Nitrozellulose transferiert (Western blot) und immunchemisch auf das Vorhandensein von Antigenen der MAK BW SCLC-1 oder -2 sowie anderer MAK bekannter Spezifität untersucht.

Hierbei wurden folgende Beobachtungen gemacht:
a) Sowohl die von MAK BW SCLC-1 als auch die von MAK BW SCLC-2 erkannten Antigene kommen in Überständen Epitop-positiver kleinzelliger Lungenkarzinomzell-Linien vor.
b) Das Molekulargewicht der Antigene liegen in der nicht reduzierenden SDS-PAGE bei 170 ± 10 kDa, 140 ± 10 kDa, 105 ± 10 kDa, 67 ± 10 kDa und 50 ± 10 kDa. Die Breite der Banden deuten auf Glykoproteine hin. Unter reduzierenden Bedingungen werden die Antigene von MAK BW SCLC-1 und -2 nicht mehr erkannt.
   Nach Immunfärbung des Western blots mit dem MAK BW SCLC-1 können nur die Antigene mit einem Molekulargewicht von 170 ± 10 kDa, 140 ± 10 kDa und 105 ± 10 kDa nachgewiesen werden.
c) Nach Behandlung der Antigene mit Vibrio cholerae Neuraminidase (0.1 U/ml für 12 Std. bei 37°C) bzw. nach Behandlung mit NaJO₄ (1 mM; 1 h, 25°C) waren beide MAK noch in der Lage, an das Antigen zu binden. diese Befunde deuten darauf hin, daß die von MAK BW SCLC-1 und -2 definierten Epitope auf den Glykoproteinantigenen Proteinepitope darstellen.
   Diese Befunde werden dadurch erhärtet, daß die Epitope durch Proteasebehandlung zerstört werden (Pronase P; 0.1 mg/ml; 72 h, 37°C).
d) Zwei im Vergleich eingesetzte MAK gegen N-CAM (neurale Zelladhäsionsmoleküle) (Kibbelaar et al., Journal of Pathology, 159: 23 - 28, 1989) zeigten beide eine Bindung an die 170 ± 10 kDa, 140 ± 10 kDa und 105 ± 10 kDa Antigene. Der MAK 735 ist gegen α-(2-8)-verknüpfte N-Acetyl-Neuraminsäure gerichtet. Es ist anzunehmen, daß es sich bei der kleineren, präferentiell gefärbten Glykoproteinbande von 105 ± 10 kDa wahrscheinlich um die kleinere der 3 iso-Formen des N-CAM handelt, die in höheren Konzentrationen neben den größeren N-CAM iso-Formen in überständen von kleinzelligen Lungenkarzinomzellinien nachweisbar sind. Die Affinitätskonstante des MAK BW SCLC-1 wurde in einem Zellbindungsassay an 3 verschiedenen humanen Gliomzelllinien bestimmt und liegt im Bereich von 1 x 10¹⁰ M⁻¹.
e) Aus Seren von SCLC-Tumorpatienten wurde mittels Affinitätschromatographie mit dem MAK BW SCLC-1 nach SDS-PAGE unter nicht reduzierenden Bedingungen zwei Antigene mit einem Molekulargewicht von 70 - 80 und 90 - 120 kDa im Western blot nachgewiesen, bei denen es sich wahrscheinlich um iso-Formen des N-CAM handelt. Desweiteren wurde aus Seren von SCLC-Patienten mittels Affinitätschromatographie mit der MAK 735-Sepharose Antigene isoliert, die unter nicht reduzierenden Bedingungen in der SDS-PAGE ein Molekulargewicht von 220 -260 kDa und 160-200 kDa aufweisen und sich im Western blot mit dem MAK BW SCLC-1 immunchemisch anfärben lassen. Im Serum gesunder Blutspender konnten unter den gleichen Versuchsbedingungen derartige Antigene nicht bzw. nur in sehr geringen Mengen nachgewiesen werden.

Anschließend wurde mittels eines Radioimmunoassays (RIA) die Anbindung des mit J-125 markierten MAK BW SCLC-1 an je 2 in vitro kultivierte humane Melanomzell- und Neuroblastomzellinien gemessen. Es wurde festgestellt, daß der MAK bei 37°C sehr schnell an Epitop-positive Zellinien band (1 - 5 min), aber relativ schnell wieder abgegeben wurde (> 10 min bei 37°C). Bei 4°C blieb der MAK lange auf den Tumorzellen gebunden. Dieser Befund und die Präsenz der 5 zuvor beschriebenen Glykoproteine (N-CAM-iso-Formen) in Überständen kleinzelliger Lungenkarzinomzellinien deuteten auf eine aktive Abgabe der Antigene durch Tumore neuroektodermalen Ursprungs hin.

Nach Biotinylierung des MAK BW SCLC-1 konnte weiterhin mittels eines Doppeldeterminantenassays gezeigt werden, daß die N-CAM Glykoproteine mindestens 2 Epitope für diesen MAK tragen.
Kompetitionsstudien mit MAK BW SCLC-2 ergaben, daß beide MAK verschiedene Epitope auf den gleichen Antigen erkennen.

### Beispiel 4

### Immunoassay zur Bestimmung des tumorassozierten Antigens in humanen Körperflüssigkeiten

Nach dem Fachmann bekannten Verfahren erfolgte die adsorptive Bindung des MAK 735 an die Polystyroloberfläche der Vertiefungen von Mikrotitrationsplatten sowie die kovalente Kopplung der MAK BW SCLC-1 und BW SCLC-2 an das Enzym Peroxidase. Zur Bestimmung der Konzentration des weiter oben beschriebenen tumorassoziierten Antigens wurden je 10 µl Probenmaterial sowie 100 µl Probenpuffer (OSND, Behringwerke) in die mit dem MAK 735 beschichteten Vertiefungen von Mikrotitrationsplatten (Nunc) pipettiert und 2 Stunden bei 37°C inkubiert.

Nach dreimaligem Waschen mit dem verdünnten Enzygnost-Waschpuffer (OSEW, BW) wurden in die einzelnen Vertiefungen je 100 µl des MAK BW SCLC-1-POD bzw. BW SCLC-2-POD-Konjugats eingefüllt. Der folgende zweistündige Inkubationsschritt bei 37°C wurde mit einem dreimaligen Waschzyklus abgeschlossen.

Für den 3. Inkubationsschritt bei Raumtemperatur wurden anschließend je 100 µl einer Puffer/ Substrat-Chromogenlösung (H₂O₂/TMB; OUVG/OUVF, BW) in die Vertiefungen pipettiert und die Enzymreaktion nach 30 min mit Enzygnost-Stopplösung (OSFA, BW) beendet. Die Extinktion der Proben wurde bei 450 nm ermittelt.

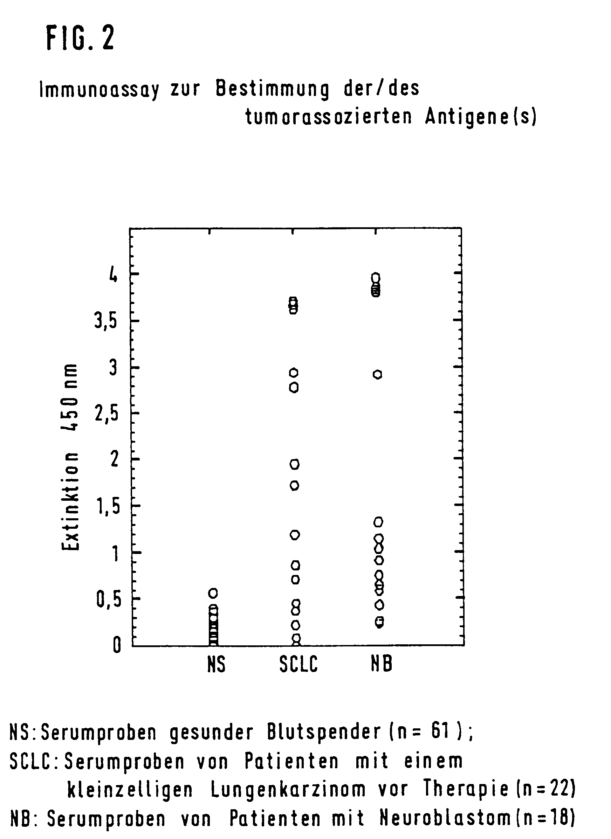

Ergebnis: Die mit diesem Immunoassay ermittelten Extinktionswerte entsprechen in ihrer Höhe der Konzentration der/des tumorassoziierten Antigene(s) in den Proben. Es zeigte sich, daß im Vergleich zu gesunden Blutspendern die Konzentration der/des tumorassoziierten Antigene(s) in den Seren von Patienten mit einem kleinzelligen Lungenkarzinom bzw. einem Neuroblastom häufig erhöht ist (Fig 2).

Auch mit anderen Testkombinationen (z. B.: Festphasenantikörper: MAK BW SCLC-1, Konjugat: wheat germ agglutinin-POD = WGA-POD; Festphasenantikörper: MAK BW SCLC-2, Konjugat: MAK BW SCLC-1-POD) konnten höhere Antigenspiegel in Tumorseren beobachtet werden. Die Unterscheidung zwischen den Serum- oder Plasmaproben von Gesunden und denen von Patienten mit malignen Tumoren war aber nicht so ausgeprägt wie mit der weiter oben beschriebenen Testvariante.

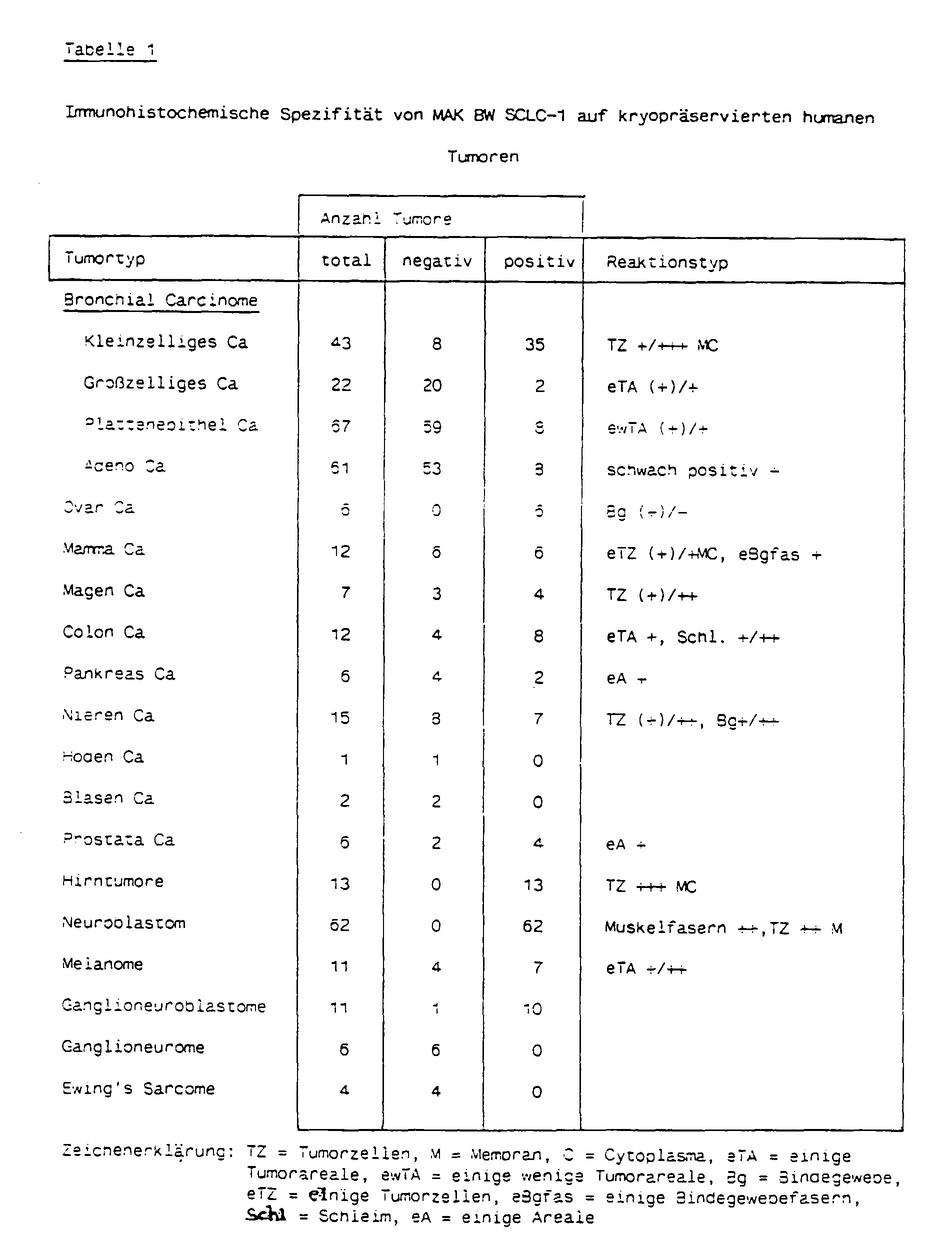

**TABELLE 2**

| **Immunhistochemische Spezifität von MAK BW SCLC-1 auf kryopräservierten humanen Normalgeweben** | | | | |
|---|---|---|---|---|
| | Anzahl Gewebe | | | |
| Gewebetyp | getestet | negativ | positiv | Reaktionstyp |
| Normalgewebe: | | | | |
| Lunge | 4 | 2 | 2 | (+) eZ ++ |
| Niere | 3 | 0 | 3 | e Gef. u. Bgfas +/++ |
| Leber | 3 | 0 | 3 | Bgfas +, e Gef.Gänge +/++ |
| | | | | |
| Magen | 2 | 0 | 2 | Bgfas +/++, Gef. ++ |
| Darm | 3 | 0 | 3 | Muskel (+) /+, Bgfas u. Gef.++ |
| | | | | |
| Pankreas | 3 | 0 | 3 | Inseln+, Bgfas u. Gef. ++ |
| | | | | |
| Prostata | 2 | 0 | 2 | Muskel +, homogen + |
| Mamma | 3 | 1 | 2 | Epithel ++, +/++ diff. |
| Gehirn | 9 | 0 | 9 | ++/+++ |
| Lymphknoten | 2 | 0 | 2 | eZ + MC |
| Knochenmark | 5 | 3 | 2 | ewZ + MC |
| Milz | 2 | 0 | 2 | (+), Gef. (+) /+ |
| Hoden | 1 | 0 | 1 | e Gänge (+) /++ |
| Blase | 1 | 0 | 1 | e Muskelfas +/++ |
| Nerven | 1 | 0 | 1 | Nervenfasern +++ |
| Mandeln | 1 | 0 | 1 | eBgfas+, e Muskelfasern +, ewZ +/++ |
| Ovar | 1 | 0 | 1 | Epithel ++, eZ Bg ++ |
| Thymus | 1 | 0 | 1 | eZ +, Hassal-Körperchen ++ |

| Zytofluorometrische Analyse auf peripheren Blutzellen | | | | |
|---|---|---|---|---|
| Lymphozyten | 2 | * 2,8 % | 0 | Zeichenerklärungen: |
| Monozyten | 2 | * 2,5 % | 0 | eZ= einige Zellen, |
| Granulozyten | 2 | * 0,8 % | 0 | e Gef.= einige Gefäße |
| Erythrozyten | 2 | * 0,2 % | 0 | Bgfas= Bindegewebefasern |
| Thrombozyten | 2 | * 0,5 % | 0 | ewZ= einige wenige Zellen e= einige, M=Membran, C= Cytoplasma |

| | | | | |
|---|---|---|---|---|
| * Anteil fluoreszierender Zellen, unter background. | | | | |

## Patentansprüche

1. Verfahren zum qualitativen Nachweis und zur quantitativen Bestimmung eines tumorassoziierten Antigens aus der Klasse der als N-CAM bezeichneten Glykoproteine in menschlichen Körperflüssigkeiten, dadurch gekennzeichnet, daß ein spezifischer Bindungspartner, der gegen Sialinsäure, Polysialinsäure oder α-(2,8)-verknüpfte N-Acetyl-Neuraminsäure gerichtet ist, in Kombination mit einem zweiten spezifischen Bindungspartner eingesetzt wird, der nicht gegen Sialinsäure, Polysialinsäure oder α-(2,8)-verknüpfte N-Acetyl-Neuraminsäure gerichtet ist, sondern ein anderes Epitop auf dem tumorassoziierten Antigen erkennt.

2. Verfahren gemäß Anspruch 1, dadurch gekennzeichnet, daß der erste spezifische Bindungspartner.ein Antikörper, ein Lectin oder ein Rezeptor ist.

3. Verfahren gemäß Anspruch 2, dadurch gekennzeichnet, daß der erste spezifische Bindungspartner der MAK 735 ist.

4. Verfahren nach einem oder mehreren der Ansprüche 1-3, dadurch gekennzeichnet, daß der zweite spezifische Bindungspartner ein Proteinepitop erkennt.

5. Verfahren nach Anspruch 4, dadurch gekennzeichnet, daß der zweite spezifische Bindungspartner der MAK BW SCLC-1 ist, der von der Hybridomzellinie (ECACC Deposit Nr. 90 022 110) produziert wird.

6. Verfahren nach Anspruch 4, dadurch gekennzeichnet, daß der zweite spezifische Bindungspartner der MAK BW SCLC-2 ist, der von der Hybridomzellinie (ECACC Deposit Nr. 90 022 109) produziert wird.

7. Verfahren nach einem der Ansprüche 1-6, dadurch gekennzeichnet, daß einer der spezifischen Bindungspartner mit einem nachweisbaren Label versehen ist.

8. Verfahren gemäß Anspruch 7, dadurch gekennzeichnet, daß das Label ein Enzym, ein Chromophor, ein Luminophor, ein Fluorophor, ein radioaktives Isotop, ein gefärbter oder ein ungefärbter Partikel ist.

9. Verfahren nach einem der Ansprüche 7 und 8, dadurch gekennzeichnet, daß der andere spezifische Bindungspartner direkt oder indirekt an eine Festphase gebunden ist.

## Claims

1. A method for the qualitative detection and for the quantitative determination of a tumor-associated antigen from the class of glycoproteins referred to as N-CAM in human body fluids, which comprises employing a specific binding partner which is directed against sialic acid, polysialic acid or α-(2,8)-linked N-acetylneuraminic acid, in combination with a second specific binding partner which is not directed against sialic acid, polysialic acid or α-(2,8)-linked N-acetylneuraminic acid but recognizes another epitope on the tumor-associated antigen.

2. The method as claimed in claim 1, wherein the first specific binding partner is an antibody, a lectin or a receptor.

3. The method as claimed in claim 2, wherein the first specific binding partner is the MAb 735.

4. The method as claimed in one or more of claims 1-3, wherein the second specific binding partner recognizes a protein epitope.

5. The method as claimed in claim 4, wherein the second specific binding partner is the MAb BW SCLC-1 which is produced by the hybridoma cell line (ECACC Deposit No. 90 022 110).

6. The method as claimed in claim 4, wherein the second specific binding partner is the MAb BW SCLC-2 which is produced by the hybridoma cell line (ECACC Deposit No. 90 022 109).

7. The method as claimed in any of claims 1-6, wherein one of the specific binding partners is provided with a detectable label.

8. The method as claimed in claim 7, wherein the label is an enzyme, a chromophore, a luminophore, a fluorophore, a radioactive isotope, a colored or an uncolored particle.

9. The method as claimed in either of claims 7 and 8, wherein the other specific binding partner is linked directly or indirectly to a solid phase.

## Revendications

1. Procédé pour la détection qualitative et la détermination quantitative d'un antigène associé à des tumeurs, appartenant à la classe des glycoprotéines désignées par N-CAM, dans des liquides corporels humains, caractérisé en ce que l'on utilise un partenaire de liaison spécifique qui est dirigé contre l'acide sialique, le poly(acide sialique) ou l'acide N-acétylneuraminique lié en α-(2,8), en association avec un second partenaire de liaison spécifique qui n'est pas dirigé contre l'acide sialique, le poly(acide sialique) ni l'acide N-acétylneuraminique lié en α-(2,8), mais reconnaît un autre épitope sur l'antigène associé à des tumeurs.

2. Procédé selon la revendication 1, caractérisé en ce que le premier partenaire de liaison spécifique est un anticorps, une lectine ou un récepteur.

3. Procédé selon la revendication 2, caractérisé en ce que le premier partenaire de liaison spécifique est l'AcM (anticorps monoclonal) 735.

4. Procédé selon une ou plusieurs des revendications 1 à 3, caractérisé en ce que le second partenaire de liaison spécifique reconnaît un épitope protéique.

5. Procédé selon la revendication 4, caractérisé en ce que le second partenaire de liaison spécifique est l'AcM BW SCLC-1, qui est produit par la lignée d'hybridomes (n° de dépôt ECACC 90 022 110).

6. Procédé selon la revendication 4, caractérisé en ce que le second partenaire de liaison spécifique est l'AcM BW SCLC-2, qui est produit par la lignée d'hybridomes (n° de dépôt ECACC 90 022 109).

7. Procédé selon l'une des revendications 1 à 6, caractérisé en ce que l'un des partenaires de liaison spécifique est muni d'un marqueur détectable.

8. Procédé selon la revendication 7, caractérisé en ce que le marqueur est une enzyme, un chromophore, un luminophore, un fluorophore, un isotope radioactif, une particule colorée ou une particule non colorée.

9. Procédé selon la revendication 7 ou 8, caractérisé en ce que l'autre partenaire de liaison spécifique est fixé directement ou indirectement à une phase solide.
